(19)

Europäisches Patentamt
European Patent Office
Office européen des brevets

(11)   EP 2 027 782 A1

(12)            EUROPEAN PATENT APPLICATION

(43) Date of publication:
    25.02.2009  Bulletin 2009/09

(51) Int Cl.:
    A23L 1/30 (2006.01)          A23L 1/305 (2006.01)
    A23L 1/05 (2006.01)          A23L 1/052 (2006.01)
    A23L 1/308 (2006.01)

(21) Application number: 07114358.0

(22) Date of filing: 15.08.2007

(84) Designated Contracting States:
    AT BE BG CH CY CZ DE DK EE ES FI FR GB GR
    HU IE IS IT LI LT LU LV MC MT NL PL PT RO SE
    SI SK TR
    Designated Extension States:
    AL BA HR MK RS

(71) Applicant: Nestec S.A.
    1800 Vevey (CH)

(72) Inventors:
    • German, Bruce
      1072 Forel (Lavaux) (CH)
    • Décombaz, Jacques
      1806 St-Légier (CH)
    • Würsch, Pierre
      1814 La Tour-de-Peilz (CH)

(74) Representative: Marquardt, Ulf
    Avenue Nestlé 55
    1800 Vevey (CH)

(54)    **Increasing the efficiency of utilization of ingested creatine**

(57)    The present invention generally relates to increasing the efficiency of usage of an ingested exogenous biologically active substance by a body. In particular, the present invention relates to ways to improve the efficiency of utilization of ingested creatine in a body, in particular to increase the storage of creatine in the body. One embodiment of the present invention is the use of a combination comprising creatine and a soluble fiber for the preparation of a composition to increase the storage of creatine in the body.

Figure 2

EP 2 027 782 A1

**Description**

The present invention generally relates to increasing the efficiency of usage of an ingested exogenous biologically active substance by a body. In particular, the present invention relates to ways to improve the efficiency of utilization of ingested creatine in a body, in particular to increase the storage of creatine in the body.

Creatine has become one of the most popular dietary supplements in the world. In 2000, the American College of Sports Medicine estimated that 2500 metric tons were consumed every year (Terjung RL, et al. Med Sci Sports Exerc 32: 706-717). Creatine improves sport performance during repeated short-duration/high-intensity exercises (Balsom PD, et al., Acta Physiol Scand 154: 303-310) and amplifies the increase in lean body mass and strength induced by resistance training (Kreider RB, et al., Med Sci Sports Exerc 30: 73-82). Creatine has also therapeutic benefits: it accelerates skeletal muscle recovery after prolonged immobilization (Hespel P, et al., J Physiol 536: 625-633) and it slows down muscle wasting in certain myopathies (Louis M, et al., Muscle Nerve 27: 604-610; Tarnopolsky MA, et al., Neurology 62: 1771-1777). Furthermore, more recent information indicates that creatine may improve cognitive performance (McMorris T, et al., Physiol Behav 90: 21-28).

A large fraction of the ingested creatine is excreted in urine: from 33 to 77% of the original dose ranging from 8g/d to 21g/d during 1 to 14 days. This might potentially induce a kidney overload. A few renal impairments have been reported (Koshy KM, et al., N Engl J Med 340: 814-815), although cohort studies have not revealed any deleterious effects on renal functions (Francaux M, et al., Int J Sports Physiol Perf 1: 309-321). Recently, increased excretion of methylamine and formaldehyde, have been observed after ingestion of large amounts of creatine (21g/d) for 2 weeks (Poortmans JR, et al., Med Sci Sports Exerc 37: 1717-1720). Although the level of these two mutagenic agents remained within the normal range for a healthy population, the development of strategies using low doses of creatine is wanted for reducing the potential risks induced by supplementation with larger amounts.

Therefore, a need exists for administering or supplying creatine in a way that its concentration in the blood of the consumer remains constant over a prolonged period of time.

There is also a need for administration of creatine in a way that prevents a "peak", that is, a transiently too high concentration of creatine in blood.

Furthermore, there is a need for consumable products that sustain an adequate concentration of creatine in blood in the range of its affectivity. In other words, the blood concentration need not only be maintained, but maintained at a useful concentration.

Finally there is a need for a way to administer creatine in way, that creatine is used by the body as efficiently as possible while avoiding that large amounts of creatine are excreted from the body unused.

Of course, if a mechanism is found that is suitable to alleviate the problems as set forth above, this mechanism must have no negative impact on the consumer and, furthermore, it preferably shouldn't interact with creatine. It would be an advantage, if a mechanism addressing these problems was based on natural substances the tolerance of which is not an issue and has not to be proven.

Overcoming these problems of the prior art and providing the art with a way to increase the efficiency of utilization of ingested creatine by the body is an object of the present invention. The present invention aims to increase the storage of ingested creatine in the body, in particular relative to the ingested creatine.

The present inventors were surprised to find that by administering creatine together with a soluble fiber they could achieve these objects of the present invention. Consequently, the object of the present invention is achieved by a use in accordance with claim 1.

An advantage of the present invention is that it prevents a high and short-term peak of creatine concentration in blood.

Another advantage of the present invention is that possible high peak entailing undesired side effects of creatine are avoided. Furthermore, the reduction of a creatine peak, e.g., in blood plasma allows a more efficient usage of the ingested creatine by the body.

Still another advantage of the present invention is that it provides a sustained concentration of creatine in blood.

Yet another advantage of the present invention is that it provides a concentration of creatine in blood plasma lying in the optimal range of effectiveness of the substance.

Importantly and unexpectedly, the present invention has the further advantage that the amount of creatine lost by excretion is minimized.

Another advantage is that many soluble fibres are known as food-components for a long time and may therefore be used without testing its tolerance in humans or animals.

A further advantage is provided by the fact that soluble fibre has a beneficial effect on the consumer. For example, it may be a prebiotic and/or it may provide energy for a prolonged time, if glucose is also present in a food product. Hence, two very different effects are achieved by using soluble fibre in a food product.

Furthermore, it is an advantage that soluble fibre does not have to be isolated in order to exert the desired effect as described above. Natural products that contain soluble fibre, for example vegetables and cereals, for example oat and barley and their bran, for example, are easily available and may directly be used.

Without wishing to be bound by theory the inventors believe that the addition of soluble fiber might slow down the velocity of intestinal creatine absorption and in addition to this also favour its retention by the body.

The present invention relates hence to the use of a combination comprising creatine and a soluble fiber for the preparation of a composition to increase the storage of creatine in the body.

The present invention also relates to the use of a combination comprising creatine and a soluble fiber for the preparation of a composition to increase the efficiency of utilization of ingested creatine.

One aspect of the present invention is the use of a combination comprising creatine and a soluble fiber for the preparation of a composition to increase the ratio of creatine stored in the body to ingested creatine.

In particular during the first 24 hours after creatine uptake the creatine loss due to excretion may be minimized. In one embodiment it is possible to minimize the creatine loss by excretion to only 5-10 %. Consequently, e.g., 24 hours after creatine ingestion a ratio of creatine stored in the body to ingested creatine of about 90% to 95% may be achieved with the use of the present invention.

In one embodiment is the use of the present invention a therapeutic use.

In another embodiment is the use of the present invention a non-therapeutic use.

With the use of the present invention creatine is stored in the body preferably in the red blood cells and/or in muscle tissue. This ensures a high bioavailability of the ingested creatine and allows a very efficient usage wherever creatine is needed in the body.

The present invention allows it to reduce the amount of creatine excretion from the body relative to the amount of creatine ingested.

The use of the present invention allows it not only to reduce the overall amount of creatine lost due to excretion but it also allows to slow down the excretion of creatine from the body. In one embodiment the use of the present invention slows down absorption rate of creatine in the gastro-intestinal tract. Short-term peaks of creatine in blood plasma are avoided and/or the blood plasma concentration of creatine is kept maintained for a prolonged time.

In an embodiment of the present invention, the food product is suitable for athletes, sportsmen and/or physically active people, because creatine is known as an ATP-recharger. A more efficient creatine usage will consequently provide a more efficient ATP-recharge system. One particular application of the subject matter of the present invention is to increase the performance of an athlete in team sports or during repeated sprints.

The compositions prepared by the use of the present invention may be administered orally or via injection. However, it is preferred that the products can be used without assistance of medical personnel and, hence, preferably the composition is a composition for oral consumption.

While the composition for oral consumption may be a medicament or a food-product it is preferred that the composition is a food product. The food product may be selected from the group consisting of bars, in particular cereal bars, cereals, in particular breakfast cereals, dried powders for reconstitution with liquids, drinks comprising fiber, or sport gels.

It is possible to produce a food product, which may be liquid or solid, comprising the combination of soluble fibre and creatine as a solid or soft bar. The composition for oral consumption may also be in the form of a cookie, a biscuit, a snack, a cracker, a bread or a bread like product. It is likewise possible to add creatine and soluble fibre to other food products, such as dairy products, ice cream, confectionery or beverages.

For example, sufficient amounts of a powder of creatine may be added to whole grain oat and/or barley flour, enriched with de-fatted oatand/or barley bran to guarantee sufficient amount of soluble fibre. This mixture may be used to form a bread dough.

If the product produced by the use of the present invention is intended to be a bar, there are a variety of possibilities to incorporate soluble fibre and creatine into a bar.

In US 4,871,557 a method for obtaining a bar with supplemental dietary fibre is disclosed. The bar according to this reference is produced by extruding a fibre mixture containing apple fibre, corn bran, water and rice flour (the latter having the function of a binder), which is afterwards particle sized, i.e. shredded or ground to obtain flakes. These flakes high in fibre are then added to the other conventional ingredients of the bar. More precisely, the fibre mixture is added to other dry ingredients as grains (e.g. toasted rolled oats or crisped rice), nuts, fruits and/or chocolate chips. These dry ingredients are mixed with the extruded-shredded fibre-flakes and a (liquid) syrup blend is added. Other ingredients may be added and the mixture is transferred to a conventional bar forming line, which subjects the mixture to a forming, heating and a cutting procedure in order to obtain a bar.

This method may easily be modified in order to obtain the composition for oral consumption according to the present invention. For example, beta-glucan may be added in the form of oat and/or barley bran concentrate to the cereals prior to extrusion. Creatine may be added at the same time or later. For example creatine may be added to the syrup.

A bar may also be prepared by mixing dry ingredients, which may be, besides soluble fibre, cereals, nuts, fruits, chocolate, berries, milk solids, for example. The dry ingredients of a bar may be later mixed with a binder, which is normally a syrup. The dry mixture will of a bar will usually comprise 40 to 90%, preferably 60 to 80% by weight of the total recipe, whereas the binder will account for the rest of it (60 to 30%, preferably 50 to 40% by weight).

Cereals may be just flour that is added to the dry mixture of the bar. However, cereals may be used in the form of crisps, flakes, puffs (e.g., oven or gun puffed), extruded and/or extruded-expanded cereals may serve as an example. Cereals are wheat, maize, barley, oat, rice, oat, millet and the like. Hence, cereals comprise, for example, rice or maize crisps, puffed rice or oat, any kind of flakes, baked or compressed and flaked cereals and so forth. Depending on the density of the final bar, the cereals may be chosen accordingly. For example, if a light bar is preferred, it is better to use crisps and/or puffs as cereals, whereas when a dense bar is preferred, it may be better to use flakes or baked and compressed cereals, or simply flour, such as rice flour, for example.

If high amounts of oat and/or barley bran and/or oat and/or barley bran concentrate (in order to provide beta-glucan) are used to prepare a dry mixture for a bar, other cereals may be completely absent.

The dry mixture may also comprise nuts and fruits, for example. Examples are hazelnuts, wall-nuts, pecan-nuts, cashew nuts, almonds, coconut, chest nut, macadamia nut or mixtures of these. Nuts may be present in amounts up to 15%, preferably up to 10% by weight of the dry mixture. Fruits, such as apple, peach, pear, apricot, banana, orange, pine-apple, for example, and/or berries, such as raspberry, strawberry, blackberry, blueberry and the like may also be added to the dry mixture.

The binder to be added to the dry mixture may be syrup. A glucose syrup, for example, comprising a mixture of glucose and/or its polymers obtained by partial hydrolysis of starch, having a DE (dextrose equivalence) value of about 30 to 50 and a water content of from about 15 to 25% by weight of the binder. Generally, the binder may comprise invert sugar, glucose sugar and/or high fructose corn syrup, for example.

The binder may comprise milk solids, which are added in the form of milk powder and/or fresh milk. In the latter case, the addition of water may be at least partially replaced by the addition of milk. WO 0056171 describes a binder in this sense, which comprises 10 to 70 parts of sugar, 0.5 to 5 parts of a binding agent (a polysaccharide such as gum), up to 15 parts of glycerin, up to 60 parts of fruit pulp or concentrate, up to 10 parts of cocoa powder and added water up to a water content of from 10 to 30%, for example.

The binder may comprise 0 to 15%, preferably 0.5 to 5% glycerol. Glycerol is sometimes added to bars to provide a moist mouth-feel, while water content has generally to remain low to grant for a prolonged shelf life.

In a basic approach, the binder is simply a syrup comprising water (10 to 20% by weight of binder) and sugars, optionally further comprising fat (0 to 15%, preferably 2 to 10%), lecithin (0 to 1%, preferably 0.01 to 0.2%) and/or flavours, which is obtained by heating the water to 70 to 90 °C and adding/dissolving the sugars under stirring. Syrups with a water content of about 15 to 20% by weight are also commercially available.

Creatine may be added to the binder, or, alternatively, it may be added together with soluble fibre to the dry mixture, for example.

By mixing the dry mixture and the binder in the amounts mentioned above, a basic mass of the bar is obtained. This step may be done in any adequate mixing apparatus such as a screw mixer of the helical spring type with an axial sprinkling nozzle or with a coating drum, for example.

The basic mass may be transferred into a suitable forming or pressing apparatus, to shape the bar. For example, a Bepex-Hutt Roller Press type DP may be used, which pushes the basic mass through a slab nozzle or a strand nozzle, under pressures of up to 12 bar, preferably 5 to 10 bar. Another suitable apparatus is the Bepex-Hutt Roller Slab Former type GP, which does not imply such high pressures and therefore yields a flat paste with a lower specific weight.

However, the final bars may be obtained by other means, too. Generally, after forming the basic mass from the binder and the dry mixture, the further process may include forming rolling, pressure rolling, transfer on a pressure band, precooling (10 to 20 °C), cutting of the final shape, for example, by a slitter or guillotine cutter (from Sollich or Rademaker), second cooling (4 to 15 DEG C), and final packaging of the bar.

For example, if the cooked cereal product according to WO 9631128 is appropriately enriched with an exogenous substance, this may be a sufficient method to obtain the food product according to the present invention.

The consumable product prepared by the use of the present invention may be any beverage enriched with creatine. For example, the liquid product may be a beverage comprising guarana powder, tea-or coffee extracts and the like.

Creatine may be added in powder form to water or another liquid or beverage, for example.

In the context of the present invention, the term "soluble fibre" means that fibre is at least 50% soluble according to the method of L. Prosky et al., J. Assoc, Off. Anal. Chem. 71, 1017-1023 (1988). For example the soluble fiber of the present invention may be selected from the group consisting of inulin, pectin, beta-glucan, gum arabic, tragacanth mucilages, guar and locust bean gum, agar, carageenans, alginates, xanthan or mixtures thereof. In one embodiment of the present invention visous soluble fibers may be used. Viscous soluble fibre may be any suitable soluble fibre, which is able to increase the viscosity of the contents of the stomach and the small intestine. Examples are beta -glucan, gums, such as guar gum, xanthan gum, and gum arabic, pectin, and, or mixtures of these. Beta -glucan is particularly preferred. As those of skill in the art will understand, the term "soluble fiber" comprises processed soluble fiber.

In another embodiment the food product according to the present invention comprises, in percent by weight, 2 to 40% of soluble fibre. Preferably, it comprises 3 to 30%, more preferably 5 to 20% of soluble fibre. For example, the food product may comprise 7 to 16% soluble fibre. Soluble fibre may be derived from any natural source and is commercially available.

Beta-glucan may be derived from any source known to contain beta-glucan. In general, also fibre rich cereal may be used. Such a cereal could also be used as a source of carbohydrate, if desired. Preferably a fibre rich cereal contains at least about 6 to 10% by weight of beta-glucan and at least about 20% to 25% by weight of total fibre. The fibre rich cereal is preferably an oat bran concentrate or a barley flour; more preferably a de-fatted oat bran or barley flour.

In this specification, a "de-fatted oat and/or barley bran concentrate" may be an oat and/or barley bran fraction which has a beta-glucan content of above 10% by weight and which has been subjected to solvent extraction to remove, at least partially, oil and fats from the fraction. Ordinarily, oat and/or barley bran concentrates have a fat or oil content of greater than about 5% by weight. De-fatted oat and/or barley bran concentrates have an oil content of less than about 7% by weight; more usually about 4% to about 6% by weight. De-fatted oat and/or barley bran concentrates offer the advantage of better stability of the food product, easier processing, and improved texture and organoleptic properties of the food product.

De-fatted oat and/or barley bran concentrates of this type are commercially available; for example suitable oat bran concentrates may be purchased from Swedish Protein AB, Vrbacka, Sweden. Barley bran concentrates are e.g. obtainable from DKSH, Asia. Alternatively the oat and/or barley bran concentrate may be prepared by grinding dry oat and/or barley grains and then carefully screening the fibre material from the starchy components of the oat and/or barley grains. The fibre rich material may then be subjected to solvent extraction techniques to remove oils and fats from the material. A suitable procedure for the extraction of oils and fats is disclosed in British patent 1,526,553; the disclosure of which is incorporated by reference. The solvent extraction step may also be carried out prior to screening if desired. This screening and extraction procedure would be suitable for producing oat and/or barley bran concentrates with fibre contents at the lower end of the range; for example an oat and/or barley bran concentrate having a maximum beta-glucan content of about 15% by weight.

In a further embodiment, the food product according to the present invention comprises, in percent by weight of the raw ingredients, 20 to 80% oat and/or barley bran concentrate. Preferably, it comprises 30 to 70%, more preferably 40 to 60% oat and/or barley bran concentrate.

In a further embodiment, the food product according to the present invention comprises, in percent by weight, 5 to 45% of protein, 1 to 20% of fats and/or oils and 40 to 80% of carbohydrates. In still a further embodiment, the food product according to the present invention comprises, in percent by weight, 5 to 20% of protein, 1 to 10% of fats and/or oils and 40 to 70% of carbohydrates. Preferably, the product comprises 10 to 15% of protein, 2 to 7% of fats and/or oils, and 50 to 60% of carbohydrates.

The protein, fats and carbohydrates may be of any plant or animal origin. For example, they may be comprised in cereals or other components used to manufacture the food product (see below), for example from oat and/or barley bran concentrate.

For optimal performance the composition prepared by the use of the present invention comprises preferably in percent by weight, about 2 to 40% of creatine, more preferably 10-35 % of creatine, even more preferred 20-30 % of creatine.

The compositions prepared by the use of the present invention are preferably ingested as follows: about 20g of creatine are ingested every day for 5 days. This will increase its muscle content by about 20%. Thereafter, about 3-5g are consumed daily to maintain the uptake of creatine by the muscle. The initial loading phase may also be omitted. In this case, the rise in muscle creatine content is slower, but reaches the same level after about 15 days. Other cell types, such as erythrocytes and neurons, also store creatine during supplementation

Accordingly, the composition prepared by the use of the present invention may be consumed in a way that about 1.5-7g, preferably about 3-5g creatine per 75kg body weight are consumed per day. Optionally, the composition may be consumed in a way that about 15-25g, preferably about 18-22g of creatine per 75kg body weight are consumed per day during the first 1-7 days.

The composition prepared by the use of the present invention may have an energy content of about 75-200 kcal, preferably or about 100-180 kcal. Preferably, the composition may be consumed in the framework of a total diet that contains about 1500-3000 kcal per day, while even more preferably 1800-2500 kcal are consumed per day. Hence, in one embodiment the energy of the composition prepared by the use of the present invention should represent about 2 - 20 %, preferably 5-15 %, even more preferred 8-12 % of the total energy consumed during a day.

It is clear to those skilled in the art that they can combine freely all features disclosed herein without departing from the scope of the invention as originally disclosed herein.

Further features and advantages of the present invention are apparent from the following examples and figures.

Figure 1 summarizes the experimental protocol.

Figure 2 presents the percentage of initial dose of creatine (2g) excreted in urine in 24h. Creatine was ingested as an aqueous solution (AS), or incorporated in a beta-glucan- (BG) or protein- (PP) rich food bar. P-values above histograms depict statistical significances of differences with AS treatment.

Figure 3 shows the kinetics of plasma creatine after an ingestion of 2g creatine. The results are expressed as the means $\pm$ SEM (n=17). *P<0.05, #P<0.01, §P<0.001 vs AS treatment same time.

Figure 4 shows the kinetics of plasma creatine after an ingestion of 2g creatine. The fitted curve is given by equation 1 (see material and method section). AS, aqueous solution; BG, beta-glucan-rich bar; PP, protein-rich bar. The results are expressed as the means $\pm$ SEM (n=17).

Figure 5 presents the plasma creatine (a) and creatinine (b) concentrations measured before (black) and after one week (grey) creatine supplementation (3 x 2g per day). P-values above histograms depict statistical significances of differences between pre- and post- creatine supplementation. AS, aqueous solution; BG, beta-glucan-rich bar; PP, protein-rich bar. The results are expressed as the means $\pm$ SEM (n=17).

Figure 6 displays the erythrocyte creatine concentration measured before (black) and after one week (grey) creatine supplementation (3 x 2g per day). P-values above histograms depict statistical significances of differences between pre- and post-creatine supplementation. AS, aqueous solution; BG, beta-glucan-rich bar; PP, protein-rich bar. The results are expressed as the means $\pm$ SEM (n=17).

**Examples**

*Subjects*

Seventeen healthy males (21 $\pm$ 0.3 years, 179 $\pm$ 1.0cm, 71 $\pm$ 2.9kg, mean $\pm$ SEM) were recruited. They were physically active, although none of them was a high level athlete. They were instructed to keep their diet and their physical activity unchanged during the experiment. Prior to the beginning of the experiment, food intake was recorded during 7 days by means of a standardized questionnaire and computed using the Nutrilog software (Marans, France). Daily energy intake was 2447 $\pm$ 111.7kcal divided into 48 $\pm$ 1.1% carbohydrates, 34 $\pm$ 1.1% lipids, 3 $\pm$ 0.8% alcohol and 15 $\pm$ 1.1% proteins which correspond to 1.3 $\pm$ 0.10g of proteins per kg body mass. The subjects did not consume any food supplement for at least 2 months and they did not take any drug on a regular basis. They were given an oral and written account of the study before signing an informed consent document. All the procedures used were in accordance with the Declaration of Helsinki.

*Protocol*

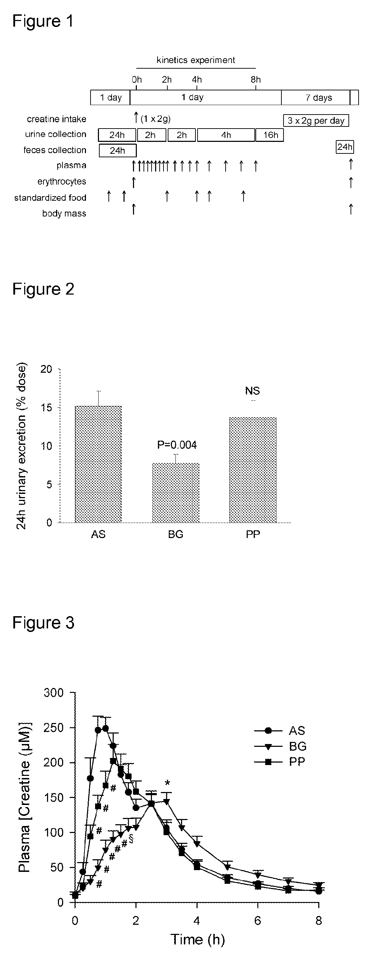

The subjects received randomly three treatments which were interspaced by a washout period of 40.0 $\pm$ 1.2 days, with a minimum of 29 days. One treatment consisted in 2g creatine powder (2.3 g creatine monohydrate, food grade, Creapure™), consumed in 150 ml of aqueous solution (AS). The two others were food bars with commercial recipes manufactured for the study by the Nestlé Product Technology Center PTC-Orbe, Switzerland: one was a protein-rich bar (PP) and the other one was a beta-glucan-rich bar (BG). The protocol is summarized in figure 1.
The BG-bar contained 16.7g carbohydrates, 4.1g proteins, 2.3g lipids and 3.2g fibers (~1.5g beta-glucans) and a total energy content of 104kcal. The PP-bar contained 19.0g carbohydrates, 14.3g proteins, 1.3g lipids and 0.1g fibers and a total energy content of 144kcal. Both bars contained also 2g of creatine, as checked by subsequent analysis, incorporated as monohydrate at manufacture.
The day before the experiment, the subjects received standardized meals; they were asked to collect their 24h urine production. Following an overnight fast, they arrived at 7.00 a.m. in the laboratory where weighed. They filled in a binary (yes/no) questionnaire assessing the occurrence of adverse symptoms over the past 7 days (stomach ache, diarrhoea, constipation, nausea, headache, dizzy spell, black out, pounding, insomnia, tendonitis, cramps, muscle soreness, fatigue, loss of appetite, hunger, other complaints). A catheter was introduced into an antecubital vein and a first blood sample was drawn to determine the basal level of creatine and creatinine in plasma and erythrocyte. Then, the subjects ingested one of the three creatine products (AS, BG or PP) and 16 blood samples were further taken up during the next 8h to determine the plasma kinetics of creatine and creatinine. A standardized breakfast, snack, lunch and a last snack were given respectively 2h, 4h, 5h and 7h after creatine ingestion. Similarly, urines were collected 2h, 4h, 8h and 24h after creatine ingestion. The subjects were instructed to consume the test product 3 times a day, in the morning, at noon and in the evening, for the next 7 days. On the eighth day, the subjects returned to the laboratory after an overnight fast. They were weighed again and a blood sample was drawn for plasma and erythrocyte creatine and creatinine determination. The health status questionnaire was filled in again. Four of the subjects collected their 24h feces on six occasions: the day before and the last day of each supplementation period.

*Procedures*

Venous blood was collected on heparin as an anticoagulant. The tubes were placed immediately on ice and centrifuged at 3000g for 6min at 4°C. Plasma (900μL) was deproteinized with sulfosalicylic acid 5% (300μL) and centrifuged again at 3000g for 6min. NaOH 0.1M (90μL) was added to supernatant (300μL) to obtain a pH value of about 7.8. Samples were stored at -20°C.

After the first centrifugation, erythrocytes were washed twice with a similar volume of physiological water (0.9% NaCl, centrifugation: 1000g, 5min, 4°C). After three frozen and defrozen procedures, erythrocytes (300μL) were deproteinized with sulfosalicylic acid 5% (600μL) and then centrifuged at 3000g during 6min. Supernatant was collected and centrifuged again. The clear supernatant was neutralized with NaOH 0.1M.

Urines were collected in a plastic bottle and the volume measured. An aliquot (10mL) was stored at -20°C.

Creatine and creatinine content in plasma, erythrocytes and urine was analyzed by spectrophotometry by enzymatic techniques ( Siedel J et al., (1984) Clin Chem 30: 968), omitting creatininase for creatine determination. Creatine and creatinine content in test bars were determined by HPLC following homogenization and solvent extraction according to a modified method of Dash and Sawhney (Dash AK, Sawhney A (2002) J Pharm Biomed Anal 29: 939-945).

Collection of 24h feces was placed in a plastic box and weighed. An aliquot was stored at -80°C. Due to the large bacterial content, the feces were prepared as soon as possible, avoiding the acidic deproteinization step that might induce a degradation of creatine to creatinine ( Ganguly S, et al., (2003) AAPS PharmSciTech 4: E25).

Both creatine and creatinine content in feces was analyzed by HPLC at 205nm. After dilution of a feces aliquot in 0.045M ammonium sulfate containing an internal standard (4-[2-aminoethyl]-benzene sulfonamide), the sample was filtered on a 10kDa membrane. The following conditions were used to identify creatine: 2 Beckman RP-C18 Ultrasphere ODS columns 5μ 25cm*4.6mm; elution: A/B (70/30) with 0.05% anhydrous trifluoroacetic acid (TFA) at 1.0mL/min; A: 0.1% $H_3PO_4$ in deionised $H_2O$; B: 1.4mg sodium dodecylsulfate/mL acetonitril. Spectral profiling (Diod Array Detector series 200 HP) demonstrated that the small peak corresponding to creatine in the samples was heterogeneous. The values reported for creatine in the results section correspond to highest possible estimates including a probable overestimation. Creatinine was detected according to another modified method ((Dash AK, Sawhney A (2002) J Pharm Biomed Anal 29: 939-945) using the same columns as for creatine but a different elution: ammonium sulfate 5.95g/L deionised $H_2O$ at 1.0mL/min. Due to the nature of the elution solvent and the small size of the creatinine peak, its spectral profile could not be obtained. As for creatine, the reported values correspond to highest possible estimates.

*Pharmacokinetics*

Using a Marquardt algorithm, all kinetics were fitted with the following equation:

$$C= \quad (FD/V*(ka/(ka-kel)) * (exp(-kel*(t-tlag))- exp(-ka*(t-tlag)))) \quad \textit{(equation 1)}$$

where

C is the plasma creatine concentration minus the basal concentration (μM) if C≤0, then data were removed
F is the bioavailability
D is the dose (2g or 15 300μmol)
V is the volume of distribution
ka is the velocity constant of absorption (first order, $h^{-1}$)
kel is the velocity constant of elimination (first order, $h^{-1}$)
t is the time (h)
tlag is the lag-time (h)

No weight was applied to data (weight = 1) and the initial parameters values were estimated using a Simplex algorithm.

This equation corresponds to a one-open compartmental model in which ka, kel and tlag are the primary parameters. Knowing their value, new kinetic parameters of plasma creatine were calculated:

$AUC_{0,\infty}$: the area under the curve integrated from zero to infinity

Since the bioavailability (F) may not be measured, only the apparent volume of distribution and the apparent clearance can be estimated:

Vd:  the apparent volume of distribution (Vd = V/F, L)
Cl:  the apparent clearance (Cl = Kel.Vd, $L.h^{-1}$)

*Statistics*

Results are presented as the means $\pm$ standard errors of the means (SEM). Differences between mean values of variables measured with the three forms of creatine ingestion (AS/BG/PP) were assessed by a mixed model representing the order of the treatments (first, second or third treatment) and when applicable, the time of measurement (pre- and post-treatment). When the variable tested was not affected by the order, either a one-way (single dose) or a two-way ANOVA (multiple doses) for repeated measures was used. When a one-way ANOVA was used, the Dunnett test was used as post-hoc test to compare BG and PP with AS as the reference treatment. In the case of a two-way ANOVA and the Bonferroni test was applied as post-hoc test to compare pre- and post-treatment conditions. Statistical significance of changes observed in frequencies of discrete variables was inferred by means of a Fisher-test. The significant threshold was set at $P < 0.05$.

Results

*Kinetics after a single dose*

*Urines*

In basal conditions, 24h urinary excretion of creatine was $2 \pm 1.9$mg. When a single dose of 2g creatine was consumed under the form of AS and PP, $15 \pm 1.9\%$ and $14 \pm 2.2\%$ of the dose, respectively, was found in urine collected during the first 24h after ingestion (Fig. 2). Less creatine was excreted after the BG treatment ($8 \pm 1.2\%$, P=0.004), as compared to AS and PP forms. Most of the excreted creatine appeared in urine during the first 2h following intake of AS (Table 1).

Table 1. Creatine urinary excretion

|  | 0-2h | 2-4h | 4-8h | 8-24h |
|---|---|---|---|---|
| AS | $0.193 \pm 0.0204$ | $0.107 \pm 0.0217$ | $0.002 \pm 0.0010$ | $0.001 \pm 0.0001$ |
| BG | $0.035 \pm 0.0087$*** | $0.108 \pm 0.0195$ | $0.010 \pm 0.0051$ | $0.001 \pm 0.0003$ |
| PP | $0.152 \pm 0.0250$ | $0.117 \pm 0.0223$ | $0.004 \pm 0.0022$ | $0.002 \pm 0.0009$ |

Urinary creatine excretion (g) during the first two hours, between the 2nd and the 4th hour, between the 4th and the 8th hour and between the 8th and the 24th hour after ingestion of 2g creatine under the form of an aqueous solution (AS), or incorporated in a beta-glucan- (BG) or protein- (PP) rich food bar. Results are expressed as the means $\pm$ SEM (n=17). ***P<0.001 vs AS treatment.
After 4h, creatine excretion values became similar to basal conditions. Creatinine urinary excretion was slightly less than 1g/24h and not affected by ingestion of 2g creatine whatever the form of ingestion. Neither creatine, nor creatinine excretions were affected by the order of the treatment.

*Plasma concentrations*

The mean basal plasma creatine concentration was $10 \pm 1.0\mu$M (Fig. 3). After ingestion of 2g creatine under the AS form, plasma concentration increased sharply to reach a peak ($299 \pm 19.5\mu$M) in less than 1 h (Table 2).

Table 2. Plasma kinetic data

|  | AS | BG | PP |
|---|---|---|---|
| Peak concentration of plasma creatine (observed, $\mu$M) | $299 \pm 19.5$ | $174 \pm 14.0$*** | $237 \pm 22.3$* |
| Time to peak concentration (observed, h) | $0.81 \pm 0.050$ | $2.40 \pm 0.177$*** | $1.31 \pm 0.146$* |

(continued)

| | AS | BG | PP |
|---|---|---|---|
| Lag-time (h) | 0.38 ± 0.033 | 0.78 ± 0.114*** | 0.43 ± 0.060 |
| Velocity constant of absorption (h⁻¹) | 23.63 ± 6.655 | 0.78 ± 0.045*** | 4.58 ± 7.050** |
| Time of absorption (h) | 0.55 ± 0.150 | 4.67 ± 0.267*** | 2.18 ± 0.385*** |
| Velocity constant of elimination (h⁻¹) | 0.68 ± 0.058 | 0.71 ± 0.056 | 0.74 ± 0.056 |
| Area under the curve (0→∞, μM·h) | 587 ± 56.2 | 501 ± 44.9 | 528 ± 57.0 |
| Apparent volume of distribution (L) | 47.1 ± 4.16 | 55.5 ± 8.88 | 60.6 ± 14.68 |
| Apparent clearance (L·h⁻¹) | 33.7 ± 6.22 | 35.4 ± 3.87 | 38.9 ± 6.70 |

Plasma kinetic data after an ingestion of 2g creatine. AS, aqueous solution; BG, beta-glucan-rich bar; PP, protein-rich bar. The results are expressed as the means ± SEM (n=17). *P<0.05, ***P<0.001 vs AS treatment.

When the same amount of creatine was consumed as a bar, plasma concentration increase was slower; maximal plasma concentration was lower and delayed (Table 2). ANOVA design for repeated measures applied to the kinetic data showed a significant interaction between time and treatments (P<0.001) indicating different time-courses according to the ingested forms (AS/BG/PP). Post-hoc analyses showed that the difference emerged during the absorption phase before the peak concentrations were reached (Fig. 3). The peak creatine concentration was lower with BG (174 ± 14.0μM) than with PP (237 ± 22.3μM, P=0.019) and the peak concentration appeared later (2.40 ± 0.177h with BG vs 1.31 ± 0.15h with PP, P<0.001).

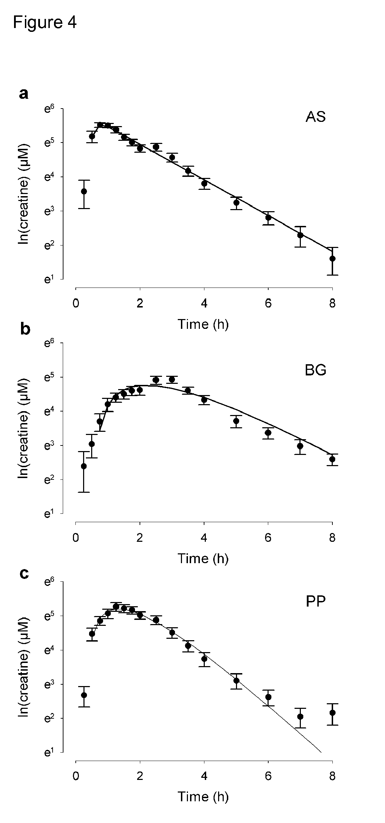

The one-open compartment model described by equation 1 yielded the best fitting of the plasma creatine time-course (Fig. 4). Other tested models did not allow the algorithm converging or gave larger residuals. Using equation 1 the median value of residuals was 1.06μM in AS condition. The first and the third quartiles were -8.90μM and 9.97μM respectively. The distributions of residuals were not different in BG and PP conditions where the medians were -0.49μM (25%: -10.04μM - 75%: 8.67μM) and 0.04μM (25%: -10.13μM - 75%: 7.98μM), respectively. The relationships between observed and calculated plasma creatine concentrations were linear and explained a high fraction of variances: 96% in AS, 88% in BG and 95% in PP condition.

The lag-time (tlag), the velocity constant of elimination (kel) and the velocity constant of absorption (ka) are the three calculated parameters of equation 1. The mixed model analysis did not reveal any effect of the order by which the treatment were given to the subjects, as far as tlag, kel and ka mean values are concerned.

The lag-time (tlag) corresponds to the time necessary to observe a change in plasma creatine concentration after the bolus ingestion. When creatine was ingested under AS and PP forms, the tlag appeared 0.38 ± 0.033h and 0.43 ± 0.060h, respectively (P=NS), but tlag doubled when creatine was ingested as a BG bar (0.78 ± 0.114h, P<0.001, Table 2)

The velocity constant of absorption (ka) was faster when creatine was ingested under the form of AS in comparison with the bars (P<0.001, Table 2). The time of absorption (Tabs) is inversely related to ka. Tabs was 0.55 ± 0.150h in AS condition whereas it reached 2.18 ± 0.385h in PP (P<0.001) and 4.67 ± 0.267h in BG condition (P<0.001).

The mean half-life of elimination was 1.09 ± 0.06h. Neither the velocity constant of elimination, nor the apparent clearance were affected by the form of creatine ingested (Table 2). The apparent volume of distribution was about 75% of body mass and was not influenced by the form of creatine ingested (Table 2). The areas under the curve calculated by integration of equation 1 from 0 to infinity ($AUC_{0,\infty}$) were identical for all creatine forms (mean value: 539 ± 30.4μM·h, Table 2).

Mean value of plasma creatinine concentrations in basal condition was 53.5 ± 1.50μM. It changed by only a few μM during the eight hours following ingestion of creatine independently of the treatment.

*Ingestion of multiple doses*

*Plasma concentrations*

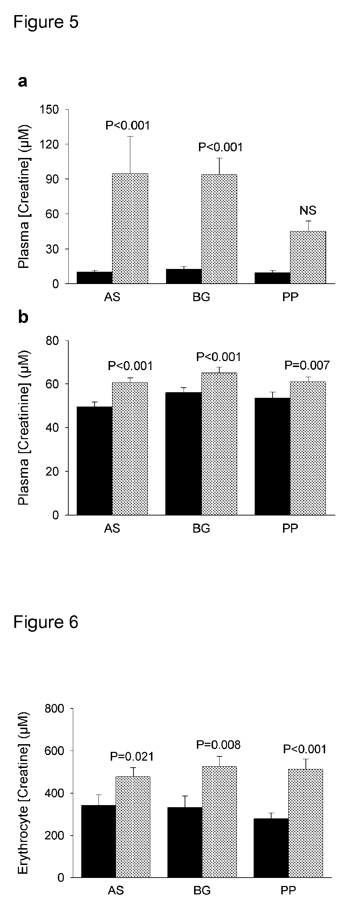

After one week creatine supplementation, plasma creatine and creatinine concentrations were measured again in a fasted state. Plasma creatine concentration, the value of which was not influenced by the order of the treatments, was 11 ± 2.5 fold higher after supplementation (Fig. 5A). The increase after PP creatine form was lower (+ 30 ± 5.2μM) than after AS and BG supplementation (+ 86 ± 33.5μM and + 85 ± 14.3μM, respectively), with a significant interaction between treatments (AS/BG/PP) and conditions (pre-/post-supplementation). There was a significant order effect for creatinine plasma concentration (P<0.001). The basal mean values were 49.0 ± 2.22 μM, 54.7 ± 3.30 μM and 56.8 ± 1.83 kg for the 1st, 2nd and 3rd trial, respectively. Despite the progressive increase in the basal concentration over the time of the experimentation, one-week creatine supplementation increased slightly plasma creatinine concentrations by all treatments (P < 0.05), independently of the form of the ingested creatine form (Fig. 5B).

*Erythrocyte concentrations*

Erythrocyte creatine concentration showed large inter-individual differences as illustrated by a coefficient of variation of basal values of 59%, but was not influenced by the order of treatments.

Erythrocyte creatine concentration increased by $88 \pm 22\%$ after creatine supplementation independently of the form of ingested creatine (Fig. 6). There was no significant interaction between treatment (AS/BG/PP) and condition (pre-/post-) indicating that the form of creatine ingested had no further influence on the erythrocyte creatine level.

Basal erythrocyte creatinine concentration was about $25\mu M$ and remained stable throughout the entire experiment.

*Feces*

Whatever the conditions, basal or supplemented, creatine and creatinine could not be quantified in human feces. The methods used were sufficiently sensitive to indicate that the maximal estimated value was $\sim 15 \pm 1.2\mu g$ per gram fresh feces for creatinine and $\sim 50 \pm 5.7\mu g$ per gram fresh feces for creatine.

*Side effects*

During the initial visit and after each period of creatine supplementation, all subjects filled in a questionnaire listing potential side effects. No difference was observed in the frequency of declared health troubles except for muscle cramps which were less frequently reported after creatine supplementation ($P<0.05$).

**Claims**

1. Use of a combination comprising creatine and a soluble fiber for the preparation of a composition to increase the storage of creatine in the body.

2. Use in accordance with claim 1 wherein creatine is stored in the red blood cells.

3. Use in accordance with one of claims 1-2 wherein creatine is stored in muscle tissue.

4. Use in accordance with one of claims 1-3 to slow down the excretion of creatine from the body.

5. Use in accordance with one of claims 1-4 to reduce the amount of creatine excretion from the body relative to the amount of creatine ingested.

6. Use in accordance with one of claims 1-5 for slowing down absorption rate of creatine in the gastro-intestinal tract.

7. Use in accordance with one of claims 1-6 to avoid short-term peaks of creatine in blood plasma and/or to keep the blood plasma concentration of creatine maintained for prolonged time.

8. Use in accordance with one of claims 1-7, wherein the composition is suitable pets or humans, in particular for athletes, sportsmen or physically active people.

9. Use in accordance with one of claims 1-8, to increase the performance of an athlete in team sports or during repeated sprints.

10. Use in accordance with one of claims 1-9, wherein the composition is a composition for oral consumption.

11. Use in accordance with claim 10, wherein the composition for oral consumption is a medicament or a food product.

12. Use in accordance with claim 11, wherein the food product is selected from the group consisting of bars, in particular cereal bars, cereals, dried powders for reconstitution with liquids, drinks comprising fiber, or sport gels.

13. Use in accordance with one of claims 1-12, wherein the soluble fiber is selected from the group consisting of inulin, pectin, beta-glucan, gum arabic, tragacanth mucilages, guar and locust bean gum, agar, carageenans, alginates, xanthan or mixtures thereof.

**14.** Use in accordance with one of claims 1-13, wherein the composition comprises, in percent by weight, about 2 to 40% of soluble fibre.

**15.** Use in accordance with one of claims 1-14, wherein the composition comprises, in percent by weight of the raw ingredients, about 20 to 80% oat and/or barley bran concentrate.

**16.** Use in accordance with one of claims 1-15, wherein the composition further comprises, in percent by weight, about 5 to 45% of protein, about 1 to 20% of fats and/or oils, and about 40 to 80% of carbohydrates.

**17.** Use in accordance with one of claims 1-16, wherein the composition comprises in percent by weight, about 2 to 40% of creatine.

**18.** Use in accordance with one of claims 1-17, wherein the composition is to be consumed in a way that about 1.5-7g creatine per 75kg body weight are consumed per day.

**19.** Use in accordance with one of claims 1-17, wherein the composition is to be consumed in a way that about 15-25g of creatine per 75kg body weight are consumed per day during the first 1-7 days and subsequently about 1.5-7g creatine per 75kg body weight are consumed per day.

**20.** Use in accordance with one of claims 1-19, wherein the composition is to be consumed in a way that a total of about 1500-3000 kcal are consumed per day.

**21.** Use in accordance with one of claims 1-20, wherein the composition has an energy content of about 75-300 kcal.

Figure 1

Figure 2

Figure 3

Figure 4

Figure 5

**a**

**b**

Figure 6

**European Patent Office**

**EUROPEAN SEARCH REPORT**

Application Number

EP 07 11 4358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/020709 A (NESTEC SA [CH]; DECOMBAZ JACQUES [CH]; MILON HUBERT [CH]; WUERSCH PIER) 10 March 2005 (2005-03-10) | 1-16 | INV. A23L1/30 A23L1/305 A23L1/05 A23L1/052 A23L1/308 |
| Y | * page 1, line 9 - line 13 * <br> * page 5, line 18 - page 7, line 14 * <br> * page 9, line 1 - page 11, line 5 * <br> * page 19, line 15 - line 24; claims 1-12 * | 17-21 | |
| Y | WO 94/02127 A (HULTMAN ERIC [SE]; HARRIS ROGER C [GB]) 3 February 1994 (1994-02-03) <br> * claims 1-18 * | 17-21 | |
| X | EP 1 362 518 A (NESTEC SA [CH]) 19 November 2003 (2003-11-19) <br> * the whole document * | 1-16 | |
| A | WO 2006/034586 A (APLODAN FORMULATIONS LTD [CA]; GARDINER PAUL T [CA]; HEUER MARVIN A [C) 6 April 2006 (2006-04-06) <br> * the whole document * | 1-21 | |
| A | US 5 908 864 A (CASEY THEODORE R [US]) 1 June 1999 (1999-06-01) <br> * the whole document * | 1-21 | TECHNICAL FIELDS SEARCHED (IPC) <br><br> A23L |
| A | DE 198 30 768 A1 (SUEDDEUTSCHE KALKSTICKSTOFF [DE]) 13 January 2000 (2000-01-13) <br> * the whole document * | 1-21 | |
| A | WO 2004/073420 A (HOWARD FOUNDATION HOLDINGS LTD [GB]; HOWARD ALAN NORMAN [GB]; HARRIS R) 2 September 2004 (2004-09-02) <br> * the whole document * | 1-21 | |

The present search report has been drawn up for all claims

| Place of search | Date of completion of the search | Examiner |
|---|---|---|
| The Hague | 22 February 2008 | FISCHER, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 07 11 4358

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2008

| Patent document cited in search report | | Publication date | Patent family member(s) | | Publication date |
|---|---|---|---|---|---|
| WO 2005020709 | A | 10-03-2005 | AU | 2003264112 A1 | 16-03-2005 |
| | | | CA | 2536828 A1 | 10-03-2005 |
| | | | US | 2007009574 A1 | 11-01-2007 |
| WO 9402127 | A | 03-02-1994 | AU | 678559 B2 | 05-06-1997 |
| | | | AU | 4594893 A | 14-02-1994 |
| | | | CA | 2140768 A1 | 03-02-1994 |
| | | | EP | 0652748 A1 | 17-05-1995 |
| | | | FI | 950302 A | 24-01-1995 |
| | | | JP | 7509230 T | 12-10-1995 |
| | | | NZ | 254307 A | 20-12-1996 |
| | | | US | 5767159 A | 16-06-1998 |
| EP 1362518 | A | 19-11-2003 | AT | 365467 T | 15-07-2007 |
| WO 2006034586 | A | 06-04-2006 | NONE | | |
| US 5908864 | A | 01-06-1999 | NONE | | |
| DE 19830768 | A1 | 13-01-2000 | NONE | | |
| WO 2004073420 | A | 02-09-2004 | AU | 2004212769 A1 | 02-09-2004 |
| | | | CA | 2514804 A1 | 02-09-2004 |
| | | | EP | 1594371 A1 | 16-11-2005 |
| | | | MX | PA05008903 A | 17-02-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4871557 A
- WO 0056171 A
- WO 9631128 A
- GB 1526553 A

**Non-patent literature cited in the description**

- **TERJUNG RL et al.** *Med Sci Sports Exerc,* vol. 32, 706-717
- **BALSOM PD et al.** *Acta Physiol Scand,* vol. 154, 303-310
- **KREIDER RB et al.** *Med Sci Sports Exerc,* vol. 30, 73-82
- **HESPEL P et al.** *J Physiol,* vol. 536, 625-633
- **LOUIS M et al.** *Muscle Nerve,* vol. 27, 604-610
- **TARNOPOLSKY MA et al.** *Neurology,* vol. 62, 1771-1777
- **MCMORRIS T et al.** *Physiol Behav,* vol. 90, 21-28
- **KOSHY KM et al.** *N Engl J Med,* vol. 340, 814-815
- **FRANCAUX M et al.** *Int J Sports Physiol Perf,* vol. 1, 309-321
- **POORTMANS JR et al.** *Med Sci Sports Exerc,* vol. 37, 1717-1720
- **L. PROSKY et al.** *J. Assoc, Off. Anal. Chem.,* 1988, vol. 71, 1017-1023
- **SIEDEL J et al.** *Clin Chem,* 1984, vol. 30, 968
- **DASH AK ; SAWHNEY A.** *J Pharm Biomed Anal,* 2002, vol. 29, 939-945
- **GANGULY S et al.** *AAPS PharmSciTech,* 2003, vol. 4, E25